# EUROPEAN PATENT SPECIFICATION

(11) **EP 0 587 106 B1**
(45) Date of publication and mention of the grant of the patent: **30.07.1997**
(21) Application number: 93114313.5
(22) Date of filing: 07.09.1993
(51) Int. Cl.: C07C 51/41, C07F 9/09, C07J 31/00, A61K 51/00, A61K 9/51

(54) **Compositions containing lipophilic salts having neutron activable cations**
Neutronenaktivierbare Kationen enthaltende lipophile Zusammensetzungen
Compositions contenant des cations activables par des neutrons

(30) Priority: 09.09.1992 IT MI922090
(43) Date of publication of application: 16.03.1994
(73) Proprietor: VECTORPHARMA INTERNATIONAL S.p.A., I-34122 Trieste (IT)
(72) Inventor: Esposito, Pierandrea, I-34135 Trieste (IT); Dobetti, Luca, I-34100 Trieste (IT); Rabaglia, Leonardo, I-43100 Parma (IT); Boltri, Luigi, I-34139 Trieste (IT)
(74) Representative: Coggi, Giorgio

(56) References cited:
- EP-A- 0 261 002
- DE-A- 1 768 940
- DE-B- 2 358 435
- US-A- 4 423 152
- GMELIN, Handbook of Inorganic Chemistry, 8th ed., part D 5, pages 88-93, 1984, Springer-Verlag, Berlin, DE
- DATABASE WPI, Week 8318, Derwent Publications Ltd., London, GB; AN 83-42327K & JP-A-58 049 307
- J. INDIAN CHEM. SOC., vol.66, no.4, 1989, pages 226-229, K.N. MEHROTRA et al
- CHEMICAL ABSTRACTS, vol. 97, no. 20, 1982, Columbus, Ohio, US; abstract no. 164925j, & SU-A-867 919
- CHEMICAL ABSTRACTS, vol. 104, no. 7, 1986, Columbus, Ohio, US; abstract no. 50555w, & JP-A-60 116 645
- CHEMICAL ABSTRACTS, vol. 102, no. 10, 1985, Columbus, Ohio, US; abstract no. 80734e
- CHEMICAL ABSTRACTS, vol. 112, no. 20, 1990, Columbus, Ohio, US; abstract no. 184657e, JP-A-1 179 764
- CHEMICAL ABSTRACTS, vol. 73, no. 26, 1970, Columbus, Ohio, US; abstract no. 136463t
- CHEMICAL ABSTRACTS, vol. 115, no. 6, 1991, Columbus, Ohio, US; abstract no. 57789m
- CHEMICAL ABSTRACTS, vol. 81, no. 5, 1974, Columbus, Ohio, US; abstract no. 26262j, JP-A-48 031 996
- CHEMICAL ABSTRACTS, vol. 109, no. 16, 1988, Columbus, Ohio, US; abstract no. 129737u
- CHEMICAL ABSTRACTS, vol. 111, no. 4, 1989, Columbus, Ohio, US; abstract no. 24545t, CN-A-87 101 021
- DATABASE WPI Week 9122, Derwent Publications Ltd., London, GB; AN 91-160053 & JP-A-03 095 292
- CHEMICAL ABSTRACTS, vol. 114, no. 10, 1991, Columbus, Ohio, US; abstract no. 93911h
- BEILSTEINS Handbuch der Organischen Chemie, 4th ed., vol. 2, 1920, Verlag von Julius Springer, Berlin, DE, page 361
- BEILSTEINS Handbuch der Organischen Chemie, 4th ed., 4th Supplement, vol. 2, part 2, 1975, Springer-Verlag, Berlin, DE, pages 986, 1019, 1043, 1069, 1118, 1130,1148, 1163, 1194, 1257, 1276
- CHEMICAL ABSTRACTS, vol. 89, no. 14, 1978, Columbus, Ohio, US; abstract no. 111430x, & JP-A-53 045 344
- CHEMICAL ABSTRACTS, vol. 102, no. 12, 1985, Columbus, Ohio, US; abstract no. 96623d, & JP-A-59 113 039
- INDIAN J. BIOCHEM. BIOPHYS., vol.25, no.3, 1988, pages 287-291, A. BANDYOPADHYAY et al
- JOURNAL OF NUCLEAR MEDICINE, vol.32, no.11, 1991, pages 2139-2143, R.J. MUMPER et al
- DIE PHARMAZIE, vol.44, no.9, 1989, pages 585-593, M. BORNSCHEIN et al

## Description

It is known that gamma scintigraphy is a technique of images acquisition for biomedical and biopharmaceutical use, applicable "in vivo" in a not invasive way.

Such technique is based on the emission of radioactivity by atoms emitting gamma radiation, selected for the favourable characteristics of energy of emission (100-300 KeV), short time of half-life, low doses of exposition for living organisms.

The use of the conventional radioisotopes, such as indium-111, technetium-99m or iodine-125, in the preparation of scintigraphic gamma radiodiagnostics, has always involved considerable disadvantages and costs, due to the manipulation of radioactive materials, and to the need of preparing the formulations in laboratories specifically equipped, and to the need of labelling, formulating and administering the radioactive label in short times, due to the isotopes half-life.

In order to overcome such disadvantages, a new technique of isotopic labelling, based on the principle of the neutron activation, was recently introduced in the gamma scintigraphy applications.

Such technique is based on the characteristic properties of some stable isotopes of originating radioisotopes emitting gamma radiation when subjected to a controlled neutron flux.

The main advantage of such technique consists of incorporating in small quantities, in the pharmaceutical form, a not radioactive isotope marker. Accordingly, all the procedures relative to the formulation can be performed by using the preferred techniques, instrumentations and times. Only the finished pharmaceutical form is subjected to the controlled neutron flux, and only after such a "bombardment" a radioactive form ready for the desired application is obtained.

Some elements, prevalently belonging to the lanthanide group, show physical and chemical characteristics such to be considered as particularly suitable to the above said radiotheraphy and radiodiagnostic applications.

In fact, short half-life times, energies of emission from 100 to 300 KeV and limited dosimetry have increased the use of elements as samarium (Sm), erbium (Er), ytterbium (Yb), in many studies regarding the in vivo behaviour of pharmaceutical forms (Digenis G.A. et al., C.R.C.-Critical Reviews in Therapeutic Drug Carrier System 7 (4), 309-345, 1991).

Most patents refer to the clinical applications of the elements activable by neutrons, and therefore they are in the specific radiotherapeutic field of the antineoplastic treatment.

EP Patent 164843, December 18, 1985, Simon J. et al., refers to complex of radionuclides of the group of rare earths with aminofosfonic acids. Complex of gadolinium (Gd), holmium (Ho), lutetium (Lu), samarium (Sm), ytterbium (Yb) are used for the selective radiotherapeutic treatment of bone tumors, reducing the radioactive exposition of the adjacent soft tissues.

Biodegradable poly(L-lactic acid) microspheres containing a complex of neutron activable Holmium are described by Russel J. Mumper et al. in the J. of Nuclear Medicine 32 (1991), No. 11. Said complex consists of Holmium acetyl-acetonate and the composition is designed for internal radiation therapy of hepatic tumors.

EP patent 176288, April 2, 1986, Simon J. et al., refers to the production of complexes analogous to those described in patent EP 164843, but complexed with aminocarboxilic organic acid, in which at least one of the aminic hydrogens is substituted by a carboxyalkyl group. Such complexes present a particularly high constant of stability (LogK=13-20), which permits the selective distribution of the radionuclide of the bone tumoral tissues.

US Patent 4898724, February 6, 1990, refers to the production of complexes of rare earths activable by neutrons, in particular Sm, and aminoalkylenphosphonic acids. Such complexes are used in veterinary for the treatment of calcific tumors.

Nevertheless, for the above applications there is the need of compounds showing more stability and lipophilicity and less solubility if compared to the complexes of the prior art.

### Summary

The authors of the present invention have found new compositions containing salts consisting of cations activable by neutrons and of lipophilic anions, having high stability and lipophilicity and low solubility, particularly suitable for the use in radiotherapeutic and radiodiagnostic applications.

Said salts are prepared by a process characterized by the following steps:
a) preparing a solution of a salt, said salt consisting of an alkalin cation and of a lipophilic anion;
b) preparing a solution of a salt, said salt consisting of a neutron activable cation and of an inorganic anion;
c) reacting the solution of step a) with the solution of step b).

Said salts are used for preparing compositions in which the same salts are supported by solid particles of suitable materials.

### Detailed description of the invention

The characteristics and the advantages of the compositions containing lipophilic salts consisting of cations activable by neutrons and of lipophilic anions according to the present invention, as well as of the process for their preparation, will be better explained in the following detailed description.

The anionic group of said salts consists of anions selected from the group consisting of C₈-C₂₂ saturated fatty acids, C₈-C₂₂ mono and poli-unsaturated fatty acids, bile acids as taurodeoxycholic acid, cholic acid, etc., C₆-C₁₈ alkylphosphonic acids, phospholipids with acid groups as diacyl-phosphatidyl-glycerols, diacyl-phosphatidic acids, cholesterolic acids, etc.

The cations of said salts comprise samarium, erbium, ytterbium, gadolidium, terbium, holmium, dysprosium, lutetium, barium and in general all the elements that, subjected to neutron irradiation, originate radionuclides emitting beta and gamma radiations.

Said lipophilic salts are obtained by preparing an aqueous solution, alcoholic or hydroalcoholic of a salt consisting of an alkalin cation and of an anion selected from the foregoing group. Then, by preparing an aqueous solution, alcoholic or hydroalcoholic of an inorganic salt selected from halide, sulfate and nitrate with a cation activable by neutrons selected from the foregoing group. Alcohols suitable for said solutions are methanol, ethanol, buthanol and isopropyl alcohol.

The former solution has a salt concentration from 0.5 mg/ml up to saturation of the solution and the latter solution has a salt concentration from 5 mg/ml up to saturation of the solution.

The two solutions are joined and the reaction is performed at a temperature in which the solvent is in the liquid-phase, preferably from 0° to 60°C, and the molar ratio of the salt with alkalin cation to the salt with cation activable by neutrons ranges from 1:0.33 to 1:2.

From said reaction the lipophilic salts of the invention are obtained. Said liphophilic salts are in a solid form, insoluble in water, slightly soluble in acid environment, and have low melting point and good workability.

The characteristics of said salts become particularly interesting when they are applied to for the preparation of compositions in which they are supported on solid particles of suitable materials.

Microparticulate polymeric materials, linear or cross-linked, of diameter lower than 200µ, biodegradable polymeric spheres of diameter lower than 100µ and lipid-base nanoparticles of diameter lower than 1µ, are employed as supporting materials or carrier systems.

The content by weight of said lipophilic salts in the composition according to the invention ranges from 0.5 to 50%. As microparticulate polymeric materials may be employed hydrophilic cross-linked polymers as polyvinyl pyrrolidone and beta-cyclodextrin, hydrophobic polymers as ethyl cellulose, methyl cellulose and Eudragit and gel hydrophilic polymers as Carbopol, xanthane, scleroglucan, hydroxypropylmethyl cellulose.

As microporous inorganic particles may be employed, for example, silica gel and aluminium sesquioxide.

As biodegradable polymeric microspheres may be employed, for example, biodegradable polyesters as lactic acid polymers, glycolic acid polymers and copolymers thereof, beta-hydroxybutirric acid polymers, poly ε-caprolactone and hyaluronic acid polysaccharides and esthers thereof.

As lipidic base nanoparticles may be employed mono-, di- and triglycerides as, for example, trilaurin, tristearin and tricapryloin, C₈-C₂₂ saturated and unsaturated fatty acids, and alcohols as, for example, stearyl alcohol, cetyl alcohol and myristic alcohol.

The techniques used for the preparation of said compositions are as follows:
- by co-milling the liphophilic salts according to the invention in mixture with said microparticulate polymer materials or with said microporose inorganic particles, with techniques at high and low energy;
- by loading said liphophilic salts on microparticulate polymeric materials of cross-linked type by controlled swelling with solutions of the above salts;
- by incorporating said salts in said lipidic base nanoparticles by producing microemulsions and following solidification;
- by incorporating said salts in biodegradable polymeric microspheres by techniques of emulsification or spray-drying;
- by incorporating said salts, in their form or loaded on lipidic or polymeric materials as above described, in polymeric films coating solid compositions, as for example, gastro-resistant or delaying the drugs diffusion, or taste masking coats.

Examples of polymeric materials for gastro-resistant coats are acrylic polymers as Eudragit^{R}, cellulosic compounds as tributyrate acetate cellulose, tri-mellitate acetate cellulose, etc.

As coats delaying diffusion examples are the RS and RL Eudragit.

Moreover, the salts according to the invention may be employed in solid compositions as, for example, those obtainable by direct compression, granulation, pelletization and spheronization, in which they have also a lubricating action.

All the preparations according to the invention may contain at least one of the active principles formulated according to the techniques known in the art in the field of the pharmaceutical formulation.

The compositions according to the invention show, when compared to the substances activable by neutrons known in the art, the advantage of a better versatility in the preparation and of a better adaptability in the internal radiotherapeutic applications as the targeted treatment of tumors, and in the radiodiagnostic applications.

The present invention is illustrated by the following examples.

### Example 1

2.0 g of sodium laurate were dissolved in 250 ml of ethylic alcohol and the mixture was stirred for 24 hours. The so obtained suspension was filtered and 10 ml of an aqueous solution containing 180 mg/ml of hexahydrate samarium chloride were added to the filtered alcoholic solution. The temperature was adjusted at 25°C and the reaction procedeed for few minutes. The precipitate was filtered, exhaustively washed before with ethylic alcohol and then with hydrochloric acid aqueous solution at pH 3.0, and stoved at 40°C under vacuum for 12 hours. The obtained samarium laurate (2.24 g) was ground and sieved at 50 mesh.

### Example 2

1.6 g of sodium stearate were dissolved in 250 ml of ethylic alcohol and then the same procedure used in Example 1 was repeated. The final product was samarium stearate (1.74 g).

### Example 3

1.8 g of sodium mirystate were dissolved in 250 ml of ethylic alcohol and then the same procedure used in Example 1 was repeated. The final product was samarium miristate (1.995 g).

### Example 4

2.0 g of sodium laurate were dissolved in 250 ml of ethylic alcohol and the mixture was stirred for 24 hours. The so obtained suspension was filtered and 10 ml of aqueous solution containing 210 mg/ml hexahydrate ytterbium chloride were added to the filtered solution. Then, the same procedure used in Example 1 was repeated. The final product was ytterbium laurate (2.31 g).

### Example 5

2.0 g of sodium laurate were dissolved in 250 ml of ethylic alcohol and the mixture was stirred for 24 hours. The so obtained suspension was filtered and 10 ml of aqueous solution containing 125 mg/ml hexahydrate erbium chloride were added to the filtered solution. Then, the same procedure used in Example 1 was repeated. The final product was erbium laurate (2.29 g).

### Example 6

500 mg of sodium taurodeoxycholate were dissolved in 2 ml of water in which 1 ml of aqueous solution containing 115 mg/ml of hexahydrate samarium cloride is poured. The precipitate was filtered, exhaustively washed with water and then stoved at 40°C under vacuum for 12 hours. The obtained samarium taurodeoxycholate (525 mg) was disgregated and sieved at 50 mesh.

### Example 7

2.0 g of monosodic phosphate C₈-ester were dissolved in 250 ml of water. Then, the same procedure used in Example 5 was repeated. The final product was samarium phosphate C₈-ester (2.23 g).

### Esempio 8

760 mg of samarium laurate prepared according to Example 1 and 6.72 g of crospovidone were weighed. The powders were milled in a planetary Fritsch mill, by agate spheres, for 1.30 hours and sieved at 50 mesh. A composition in which samarium laurate was 10.1% by weight was obtained.

### Esempio 9

610 mg of ytterbium laurate prepared according to Example 4 and 5.35 g of crospovidone were weighed. Then, the same procedure used in Example 8 was repeated. A composition in which ytterbium laurate was 10.2 % by weight was obtained.

### Example 10

600 mg of erbium laurate prepared according to Example 5 and 5.25 g of crospovidone were weighed. Then, the same procedure used in Example 8 was repeated. A composition in which erbium laurate was 10.3 % by weight was obtained.

### Example 11

740 mg of samarium laurate prepared according to Example 1 and 6.47 g of ethyl cellulose were weighed. The powders were milled in a planetary Fritsch mill, by agate spheres, for 2 hours and sieved at 50 mesh. A composition in which samarium laurate was 10.3 % by weight was obtained.

### Example 12

735 mg of ytterbium laurate prepared according to Example 4 and 6.72 g of ethyl cellulose were weighed. Then, the same procedure used in Example 11 was repeated. A composition in which ytterbium laurate was 9.9 % by weight was obtained.

### Example 13

750 mg of erbium laurate prepared according to Example 5 and 6.85 g of ethyl cellulose were weighed. Then, the same procedure used in Example 11 was repeated. A composition in which erbium laurate was 9.9 % by weight was obtained.

### Example 14

1.19 g of samarium stearate prepared according to Example 2 and 6.80 g of ethyl cellulose were weighed. Then, the same procedure used in Example 11 was repeated. A composition in which samarium stearate was 14.9 % by weight was obtained.

### Example 15

10 mg of samarium laurate prepared according to Example 1 were dissolved in 200 mg lauric acid melted at 70°C. The oily-phase was emulsified with 1 ml of water containing 130 mg of sodium taurodeoxycholate, 200 ml of Tween 80 and 100 ml of butyric acid. The microemulsion was dispersed in water at 4°C, with consequent solidification of the lipidic nanospheres, which were then washed by ultrafiltration and lyophilized. A composition in which samarium laurate was 4.8 % by weight was obtained.

### Example 16

10 mg of samarium taurodeoxycholate prepared according to Example 6 were dissolved in 200 mg lauric acid melted at 70°C. Then, the same procedure used in Example 15 was repeated. A composition in which samarium taurodeoxycholate was 4.8 % by weight was obtained.

### Example 17

The following components were weighed:

| | |
|---|---|
| Microcrystalline cellulose | 20 mg |
| Starch | 104 mg |
| SD lactose | 34 mg |
| Silica | 2 mg |
| Samarium stearate | 2 mg |

Cellulose, starch and lactose were sieved at 35 mesh and then mixed with a rotating mixer for 20 minutes. At the end, the samarium stearate and silica, in advance sieved at 40 mesh, were added. The mixing was completed in a rotating mixer for additional 10 minutes. The mixture was compressed by a rotating compressing device by using moulds (8 mm of diameter), applying a pressure of 1000 Kg/cm².

### Example 18

The following components were weighed:

| | |
|---|---|
| Crospovidone | 30 mg |
| Starch | 20 mg |
| SD lactose | 187 mg |
| Silica | 3 mg |
| Samarium laurate | 3 mg |

Crospovidone, starch and lactose were sieved at 35 mesh and then mixed with a rotating mixer for 20 minutes. At the end, the samarium laurate and silica, in advance sieved at 40 mesh, were added. The mixing was completed in a rotating mixer for additional 10 minutes. The mixture was compressed by a rotating compressing device by using moulds (10 mm of diameter), applying a pressure of 1500 Kg/cm².

### Example 19

The following components were weighed:

| | |
|---|---|
| Microcrystalline cellulose | 30 mg |
| Starch STA-RX 1500 | 20 mg |
| SD lactose | 139.5 mg |
| Silica | 3 mg |
| Carboxymethyl sodium starch | 25 mg |
| Ytterbium stearate | 3.5 mg |
| Total | 221 mg |

Cellulose, starch and lactose were sieved at 35 mesh and then mixed with a rotating mixer for 20 minutes. At the end, the ytterbium stearate and silica, in advance sieved at 40 mesh, were added. The mixing was completed in a rotating mixer for additional 10 minutes. The mixture was compressed by a rotating compressing device by using moulds (8 mm of diameter), applying a pressure of 2000 Kg/cm².

The so obtained tablets, having a surface of 1.4 cm², were then coated with a gastroresistent polymer.

The coating formulation is as follows:

| | |
|---|---|
| Metacrilic polymer acid (Eudragit L 30D) | 43.00 g (dried 12.90) |
| Samarium laurate | 5.11 g |
| Triacetin | 1.29 g |
| Water | 50.60 g |
| Total | 100.00 g |

A cycle of coating of the tablets was carried out using 100 g of suspension of the described formulation for 500 g of tablets, in a fluid bed film coating device "Glatt" GPCG 1 equipped with Wurster insert. The suspension amount was such to permit the supply of 3.5 mg of polymer on cm² of surface of coating and 2 mg of samarium laurate for each tablet.

### Example 20

600 mg of poly(lactic-co-glycholic) acid (PLG 75/25) were dissolved in 12 ml of a mixture 8/2 v/v of methylenchloride/ethanol with 5% polyethylenglychol 2000 and 0.1% Tween 80 (percentages by weight calculated compared with the PLG polymer). 30 mg of samarium laurate were dissolved in the solution and the resultant solution were emulsified in 400 ml of an aqueous solution at 0.1% of Tween 80. Stirring was maintained until complete evaporation of the solvent and the so obtained particles were centrifuged, filtered and dried under vacuum. The sizes of the obtained product ranges from 10 to 30 µm.

### Example 21

2.5 g of poly(lactic) acid (PLA) were dissolved in a mixture 9/1 v/v of methylenchloride/ethanol with 5% polyethylenglychol 4000 and 1.5 % Tween 60 (percentages by weight calculated compared with the polymer). 50 mg of samarium taurodeoxycholate were dissolved in the solution and the resultant solution were spray dried. The sizes of the obtained particles ranges from 20 to 50 µm.

### Solubility tests of lipophilic salts and the composition thereof according to the invention

The lipophilic salts and the composition thereof, obtained as described in the foregoing examples, were used for solubility tests and the results are reported in tables 1-3.

In table 1 the solubility values at 24 hours of samarium and ytterbium lipophilic salts at different pH were reported. The solubility was determined via complex-formation, using as complex agent xylelol orange (the minimum amount detectable corresponds to a solubility of 2 µg/ml). Said salts show a low solubility at pH 1.2, of order of magnitude lower of that of the respective oxyde and hydroxyde at the same pH, are pratically insoluble at pH 3.0 and totally insoluble at pH>3.

**TABLE 1**

| Lipophilic salt | Solubility (µg/ml) | | | |
|---|---|---|---|---|
| | pH=1.2 | pH=3.0 | pH=5.2 | pH=7.0 |
| Samarium laurate | 220 | 47 | <2 | <2 |
| Samarium miristate | 90 | 8 | <2 | <2 |
| Samarium stereate | 100 | 7 | <2 | <2 |
| Ytterbium laurate | 430 | 56 | <2 | <2 |

In table 2 the solubility values at 24 hours of the compositions prepared by co-milling samarium laurate (SmL) and ytterbium laurate (YbL) with crospovidone (PVP) and ethyl cellulose (EC) at pH 1.2. The solubility of said salts in said composition decreases drastically compared with the salts as they are, and in more marked way with the salts co-milled with ethyl cellulose.

**TABLE 2**

| Co-milling Hours | Solubility (µg/ml) | | | |
|---|---|---|---|---|
| | EC/YbL | PVP/YbL | EC/SmL | PVP/SmL |
| 0 | 430 | 430 | 220 | 220 |
| 0.30 | - | - | - | 49 |
| 1 | - | 160 | 26 | 47 |
| 1.30 | - | - | 24 | 43 |
| 2 | 39 | - | 17 | - |

In table 3 the solubility values at 24 hours of the compositions prepared by co-milling of samarium laurate (SmL) and ytterbium laurate (YbL) with crospovidone (PVP) and ethyl cellulose (EC) at pH 3.0. The solubility of said salts in said composition decreases drastically compared with the salts as they are, and in a more marked way with the salts co-milled with ethyl cellulose. In fact, it can be noted that the samarium laurate co-milled with ethyl cellulose is totally insoluble already after 1.30 hours of co-milling.

**TABLE 3**

| Co-milling Hours | Solubility (µg/ml) | | | |
|---|---|---|---|---|
| | EC/YbL | PVP/YbL | EC/SmL | PVP/SmL |
| 0 | 56 | 56 | 47 | 47 |
| 0.30 | - | - | - | 31 |
| 1 | - | 8 | 7 | 34 |
| 1.30 | - | - | <2 | 13 |
| 2 | 13 | - | <2 | - |

## Claims

1. Compositions comprising lipophilic salts and a solid supporting material characterized in that said lipophilic salts consist of cations activable by neutrons and of lipophilic anions and said supporting material is selected from the group consisting of microparticles of linear or cross-linked polymeric materials, microporose inorganic particles selected from the group consisting of silica gel and aluminium sesquioxide, biodegradable polymeric microspheres and lipidic base nanoparticles.

2. Compositions according to claim 1, characterized in that said cations are selected from the group consisting of samarium, erbium, ytterbium, gadolinium, terbium, holmium, dysprosium, lutetium and barium.

3. Compositions according to claim 1, characterized in that said anions are selected from the group of anions derived from C₈-C₂₂ saturated fatty acids, C₈-C₂₂ mono- and polyunsaturated fatty acids, bile acids, alkylphosphonic acids with C₆-C₁₈ alkyl chain, phospholipids with acid groups, diacylphosphatidic acids and cholesterolic acids.

4. Compositions according to claim 1, characterized in that the content of said lipophilic salts ranges from 0.5 to 50% by weight.

5. Compositions according to claim 1, characterized in that said microparticles of polymeric materials have a diameter size lower than 200 µ, said microporose inorganic particles have a diameter size lower than 200 µ, said biodegradable polymeric microspheres have a diameter size lower than 100 µ and said lipidic base nanoparticles have a diameter lower than 1 µ.

6. Compositions according to claim 1, characterized in that said microparticles of polymeric materials are selected from the group consisting of polyvinyl pyrrolidone, beta-cyclodextrin, ethyl cellulose, methyl cellulose, Eudragit, Carbopol, xanthane, scleroglucan and hydroxypropylmethyl cellulose.

7. Compositions according to claim 1, characterized in that said biodegradable polymeric microspheres are selected from the group consisting of lactic acid polymers, glycolic acid polymers and copolymers thereof, beta-hydroxybutirric acid polymers, poly ε-caprolactone and hyaluronic acid polysaccharides and esters thereof.

8. Compositions according to claim 1, characterized in that said lipidic base nanoparticles are selected from the group consisting of trilaurin, tristearin, tricapryloin, C₈-C₂₂ saturated and unsaturated fatty acids, stearyl alcohol, cetyl alcohol and myristic alcohol.

9. Compositions according to claim 1, characterized in that when said supporting material consists of microparticles of polymeric materials and of microporose inorganic particles, they are prepared by co-milling techniques at high and low energy.

10. Compositions according to claim 1, characterized in that when said supporting material consists of lipidic base nanoparticles, they are prepared by producing microemulsions and following solidification.

11. Compositions according to claim 1, characterized in that when said supporting material consists of biodegradable polymeric microspheres, they are prepared by techniques of emulsification.

12. Compositions according to claim 1, characterized in that when said supporting material consists of biodegradable polymeric microspheres, they are prepared by techniques of spray-drying.

## Patentansprüche

1. Zusammensetzungen, umfassend lipophile Salze und ein festes Trägermaterial,
dadurch **gekennzeichnet**, daß
die lipophilen Salze aus Kationen, die durch Neutronen aktivierbar sind, und lipophilen Anionen bestehen und daß das Trägermaterial aus der Gruppe ausgewählt ist, bestehend aus Mikroteilchen von linearen oder vernetzten Polymermaterialien, mikroporösen, anorganischen Teilchen, ausgewählt aus der Grupe, bestehend aus Silicagel und Aluminiunsesquioxid, bioabbaubaren, polymeren Mikrokugeln und Nanoteilchen auf Lipidbasis.

2. Zusammensetzungen nach Anspruch 1,
dadurch **gekennzeichnet**, daß
die Kationen aus der Gruppe ausgewählt sind, bestehend aus Samarium, Erbium, Ytterbium, Gadolinium, Terbium, Holmium, Dysprosium, Lutetium und Barium.

3. Zusammensetzungen nach Anspruch 1,
dadurch **gekennzeichnet**, daß
die Anionen ausgewählt sind aus der Gruppe aus Anionen, die sich von gesättigten C₈₋₂₂-Fettsäuren, mono- und polyungesättigten C₈₋₂₂-Fettsäuren, Gallensäuren, Alkylphosphonsäuren mit C₆₋₁₈-Alkylkette, Phospholipiden mit sauren Gruppen, Diacylphosphatidinsäuren und Cholesterolsäuren ableiten.

4. Zusammensetzungen nach Anspruch 1,
dadurch **gekennzeichnet**, daß
der Gehalt der lipophilen Salzen in dem Bereich von 0,5 bis 50 Gew.% liegt.

5. Zusammensetzungen nach Anspruch 1,
dadurch **gekennzeichnet**, daß
die Mikroteilchen der polymeren Materialien eine Durchmessergröße von weniger als 200 µ, die mikroposösen, anorganischen Teilchen eine Durchmessergröße von weniger als 200 µ, die bioabbaubaren, polymeren Mikrokugeln eine Durchmessergröße von weniger als 100 µ und die Nanopartikel auf Lipidbasis einen Durchmesser von weniger als 1 µ haben.

6. Zusammensetzungen nach Anspruch 1,
dadurch **gekennzeichnet**, daß
die Mikroteilchen aus polymeren Materialien ausgewählt sind aus der Gruppe, bestehend aus Polyvinylpyrrolidon, β-Cyclodextrin, Ethylcellulose, Methylcellulose, Eudragit, Carbopol, Xanthan, Scleroglucan und Hydroxypropylmethylcellulose.

7. Zusammensetzungen nach Anspruch 1,
dadurch **gekennzeichnet**, daß
die bioabbaubaren, polymeren Mikrokugeln ausgewählt sind aus der Gruppe, bestehend aus Milchsäurepolymeren, Glycolsäurepolymeren und Copolymeren davon, β-Hydroxybuttersäurepolymeren, Poly-ε-Caprolaton und Hyaluronsäurepolysacchariden und Estern davon.

8. Zusammensetzungen nach Anspruch 1,
dadurch **gekennzeichnet,** daß
die Nanopartikel auf Lipidbasis ausgewählt sind aus der Grupe, bestehend aus Trilaurin, Tristearin, Tricapryloin, gesättigten und ungesättigten C₈₋₂₂-Fettsäuren, Stearylalkohol, Cetylalkohol und Myristylalkohol.

9. Zusammensetzungen nach Anspruch 1,
dadurch **gekennzeichnet**, daß
dann, wenn das Trägermatrial aus Mikroteilchen aus polymeren Materialien und aus mikroporösen, anorganischen Teilchen besteht, diese durch gemeinsame Mahltechniken mit niedriger und hoher Energie hergestellt sind.

10. Zusammensetzungen nach Anspruch 1,
dadurch **gekennzeichnet**, daß
dann, wenn das Trägermaterial aus Nanoteilchen auf Lipidbasis besteht, diese durch Erzeugung von Mikroemulsionen und anschließender Verfestigung hergestellt sind.

11. Zusammensetzungen nach Anspruch 1,
dadurch **gekennzeichnet**, daß
dann, wenn das Trägermaterial aus bioabbaubaren, polymeren Mikrokugeln besteht, diese durch Emulsionstechniken hergestellt sind.

12. Zusammensetzungen nach Anspruch 1,
dadurch **gekennzeichnet,** daß
dann, wenn das Trägermaterial aus bioabbaubaren, polymeren Mikrokugeln besteht, diese durch Sprühtrocknungstechniken hergestellt sind.

## Revendications

1. Compositions comprenant des sels lipophiles et un matériau support solide, caractérisées en ce que lesdits sels lipophiles consistent en cations activables par des neutrons, et en anions lipophiles, et que ledit matériau support est choisi au sein du groupe formé par des microparticules de matériaux polymère linéaires ou réticulés, des particules minérales microporeuses choisies au sein du groupe comprenant le gel de silice et le sesquioxyde d'aluminium, des microsphères polymère biodégradables, et des nanoparticules de bases lipidiques.

2. Compositions selon la revendication 1, caractérisées en ce que lesdits cations sont choisis au sein du groupe formé par le samarium, l'erbium, l'ytterbium, le gadolinium, le terbium, l'holmium, le dysprosium, le lutécium, et le baryum.

3. Compositions selon la revendication 1, caractérisées en ce que lesdits anions sont choisis au sein du groupe des anions dérivés d'acides gras saturés en C₈-C₂₂, d'acides gras mono- et poly-insaturés en C₈-C₂₂, d'acides biliaires, d'acides alkylphosphoniques à chaîne alkyle C₆-C₁₈, de phospholipides comportant des groupes acide, d'acides diacylphosphatidiques, et d'acides cholestéroliques.

4. Compositions selon la revendication 1, caractérisées en ce que la teneur desdits sels lipophiles est comprise entre 0,5 et 50 % en poids.

5. Compositions selon la revendication 1, caractérisées en ce que lesdites microparticules de matériaux polymère ont un diamètre inférieur à 200 µm, lesdites particules minérales, microporeuses ont un diamètre inférieur à 200 µm, lesdites microsphéres polymère biodégradables ont un diamètre inférieur à 100 µm, et lesdites nanoparticules de bases lipidiques ont un diamètre inférieur à 1 µm.

6. Compositions selon la revendication 1, caractérisées en ce que lesdites microparticules de matériaux polymère sont choisies au sein du groupe comprenant la polyvinylpyrrolidone, la β-cyclodextrine, l'éthylcellulose, la méthylcellulose, l'Eudragit, le Carbopol, le xanthane, le scléroglucan, et l'hydroxypropylméthylcellulose.

7. Compositions selon la revendication 1, caractérisées en ce que lesdites microsphères polymère biodégradables sont choisies au sein du groupe comprenant les polymères d'acide lactique, les polymères d'acide glycolique, et les copolymères de ceux-ci, les polymères d'acide β-hydroxybutyrique, les polysaccharides de poly-ε-caprolactone et d'acide hyaluronique, et les esters de ceux-ci.

8. Compositions selon la revendication 1, caractérisées en ce que lesdites nanoparticules de bases lipidiques sont choisies au sein du groupe comprenant la trilaurine, la tristéarine, le tricapryloïne, les acides gras C₈-C₂₂, saturés et insaturés, l'alcool stéarylique, l'alcool cétylique, et l'alcool myristique.

9. Compositions selon la revendication 1, caractérisées en ce que ledit matériau support consiste en microparticules de matériaux polymère, et en particules minérales microporeuses qui sont préparées par des techniques de co-broyage à forte et à faible énergie.

10. Compositions selon la revendication 1, caractérisées en ce que ledit matériau support consiste en nanoparticules de bases lipidiques qui sont préparées en produisant des émulsions suivies de solidification.

11. Compositions selon la revendication 1, caractérisées en ce que ledit matériau support consiste en microsphères polymère biodégradables qui sont préparées par des techniques d'émulsification.

12. Compositions selon la revendication 1, caractérisées en ce que ledit matériau support consiste en microsphères polymère biodégradables qui sont préparées par des techniques d'atomisation.
